# EUROPÄISCHE PATENTSCHRIFT

(11) **EP 2 662 100 B1**
(45) Veröffentlichungstag und Bekanntmachung des Hinweises auf die Patenterteilung: **13.08.2014**
(21) Anmeldenummer: 13165737.1
(22) Anmeldetag: 29.04.2013
(51) Int. Cl.: A61M 1/14, B01D 61/30, B25B 5/14

(54) **Filterhalter; medizinisches Gerät zur extrakorporalen Blutbehandlung mit Filterhalter**
Filter holder; medical device for extracorporeal blood treatment with filter support
Porte-filtre, appareil médical pour le traitement extracorporel du sang avec porte-filtre

(30) Priorität: 11.05.2012 DE 102012104157
(43) Veröffentlichungstag der Anmeldung: 13.11.2013
(73) Patentinhaber: B. Braun Avitum AG, 34212 Melsungen (DE)
(72) Erfinder: Schade, Andreas, 36199 Rotenburg (DE)
(74) Vertreter: Winter, Brandl, Fürniss, Hübner, Röss, Kaiser, Polte - Partnerschaft

(56) Entgegenhaltungen:
- DE-A1- 4 206 605
- US-A- 5 824 213
- US-A1- 2003 120 327
- US-A1- 2005 118 059
- US-A1- 2007 252 057

## Beschreibung

Die Erfindung betrifft einen Filterhalter zur Halterung eines Filters am Gehäuse eines medizinischen Geräts zur extrakorporalen Blutbehandlung, wobei der Filterhalter wenigstens einen ersten Greifschenkel und einen zweiten Greifschenkel aufweist, die so zueinander angeordnet sind, dass zwischen den beiden Greifschenkeln ein Aufnahmebereich gebildet ist, in welchen der Filter einbringbar ist.

Die Erfindung betrifft ferner ein medizinisches Geräts zur extrakorporalen Blutbehandlung mit einem solchen Filterhalter.

Bei Medizingeräten mit extrakorporalem Blutkreislauf zur Blutreinigung werden üblicherweise Blutschlauchsysteme an dem Medizingerät angebracht. Zur Orientierung und Sortierung sind hierbei Schlauchhalter hilfreich. Unter anderem werden auch im Bereich des Dialysatorhalters Schlauchhalter benötigt, die insbesondere ein Abknicken der Schläuche zum/vom Dialysator verhindern sollen.

Dabei ist es bekannt, die Schlauchhalterfunktion durch lösbare Anbauteile beispielsweise am Gehäuse des medizinisches Geräts zu realisieren. Lösbare Anbauteile sind dann jedoch auch verlierbar, was nachteilig ist. Fest montierte Anbauteile mit Schlauchhaltern können dagegen abbrechen, was ebenfalls nachteilig sein kann. Ferner kann die Anordnung der Schlauchhalter unbefriedigend sein, wenn Schlauchhalter in einem bestimmten Bereich wie dem Dialysator benötigt werden, ohne dass hierfür geeignete Positionen gegeben sind.

Weiter ist beispielsweise aus der DE 24 12 285 A1 ein Drehwechselhalter ohne Schlauchhalterfunktion bekannt, der dem Anschluss von künstlichen Nieren dient. Mit dem beschriebenen Drehwechselhalter kann eine künstliche Niere während des Gebrauchs um 180° gedreht werden, ohne dabei Schläuche, die an die künstliche Niere angeschlossen sind abzuknicken.

Eine weiterer Filterhalter ohne Schlauchhalterfunktion ist aus der DE 10 2009 024 448 A1 bekannt. Weitere Filterhalter gemäß dem Oberbegriff von Anspruch 1 sind bekannt aus US 2007/0252057 A1 und US 0058244213 A.

Ein medizinisches Gerät zur extrakorporalen Blutbehandlung gemäß dem Oberbegriff von Anspruch 10 ist auch bekannt aus US 2005/0118059 A1.

Schließlich ist aus der WO 2011/073766 A1 ein Halteelement für Schlauch-Spannvorrichtungen in medizinischen Vorrichtungen bekannt.

Aufgabe der Erfindung ist es daher, einen robusten Filterhalter bereitzustellen, mit dem Schläuche im Bereich eines Filters gehalten werden können.

Aufgabe der Erfindung ist es ferner, ein medizinisches Gerät mit einem solchen Filterhalter bereitzustellen.

Erfindungsgemäß wird diese Aufgabe durch einen Filterhalter gemäß dem unabhängigen Anspruch 1 gelöst. Vorteilhafte Weiterbildungen des Filterhalters ergeben sich aus den Unteransprüchen 2-9. Die Aufgabe wird ferner durch ein medizinisches Gerät zur extrakorporalen Blutbehandlung gemäß Anspruch 10 gelöst.

Der erfindungsgemäße Filterhalter dient zur Halterung eines Filters am Gehäuse eines medizinischen Geräts zur extrakorporalen Blutbehandlung, wobei der Filterhalter wenigstens einen ersten Greifschenkel und einen zweiten Greifschenkel aufweist, die so zueinander angeordnet sind, dass zwischen den beiden Greifschenkeln ein Aufnahmebereich gebildet ist. Der Filter kann dann so in diesen Aufnahmebereich zwischen den beiden Greifschenkeln eingebracht werden, dass der Filter wenigstens gegen die Schwerkraft im Filterhalter gehalten wird.

Erfindungsgemäß ist an wenigstens einem Greifschenkel wenigstens eine rinnenförmige Einbuchtung vorgesehen, deren Querschnitt das Einklemmen eines Schlauchs ermöglicht. Diese wenigstens eine Einbuchtung bildet somit am Filterhalter einen Schlauchhalter aus. Hierdurch erfüllt der Filterhalter neben der Funktion eines Filterhalters auch das Erfordernis eines Schlauchhalters im Bereich des Filters, wobei keine zusätzlichen Bauteile erforderlich sind. Der Schlauchhalter ist vielmehr in die Filterhaltergeometrie integriert und kann kostenneutral abgebildet werden.

Ferner handelt es sich um eine einteilige Lösung, die keinerlei Schnittstellen zu anderen Bauteilen erfordert. Dies erhöht auch die Patientensicherheit, da der Schlauchhalter unverlierbar am Filterhalter angebracht ist und Schläuche sehr nah am Filter sortiert und orientiert werden können. Der wenigstens eine Schlauchhalter ist dabei in das Erscheinungsbild der Greifschenkel des Filterhalters integriert und stört daher nicht die Gesamterscheinung.

Vorzugsweise befindet sich die wenigstens eine rinnenförmige Einbuchtung an einer Außenseite des Greifschenkels, die von dem Aufnahmebereich weg gerichtet ist. Der Schlauchhalter ist in diesem Fall außen am Filterhalter ausgebildet. Er könnte jedoch auch an einer Innenseite des Filterhalters ausgeformt sein, so dass ein oder mehrere Schläuche auch in einen Bereich zwischen den beiden Greifschenkeln eingebracht werden können, falls dies bei bestimmten Anwendungen vorteilhaft ist und dort ausreichend Raum vorhanden ist.

In einem Ausführungsbeispiel der Erfindung sind an dem wenigstens einen Greifschenkel zwei rinnenförmige Einbuchtungen vorgesehen, so dass zwei Schlauchhalter ausgeformt sind. Auch eine höhere Anzahl von Einbuchtungen ist möglich. Ferner kann wenigstens eine rinnenförmige Einbuchtung quer zur Längsausrichtung eines Greifelements verlaufen. Dies führt dann typischerweise zu einer vertikalen Orientierung eines Schlauches, wenn die Greifschenkel horizontal verlaufen. Ein oder mehrere Schlauchhalter können jedoch auch in andere oder sogar unterschiedliche Richtungen ausgerichtet sein. So können Schläuche bei Bedarf auch in unterschiedliche Richtungen geführt werden. Darüber hinaus können die Einbuchtungen der Schlauchhalter auch verschiedene Querschnitte aufweisen, so dass unterschiedlich große Schläuche eingeklemmt werden können.

Der wenigstens eine Greifschenkel kann aus einem Metallprofil gebildet sein, wie es oftmals für Filter im Bereich der extrakorporalen Blutbehandlung eingesetzt wird. Insbesondere kann es sich um ein Strangpressprofil beispielsweise aus Aluminium handeln.

Um einen Filter im Aufnahmebereich zwischen den beiden Greifschenkeln aufnehmen und halten zu können, können die Geometrie und die Mechanik des Filterhalters beliebig ausgestaltet sein. In einem bevorzugten Ausführungsbeispiel der Erfindung ist der erste Greifschenkel drehbar an dem zweiten Greifschenkel gelagert, so dass die beiden Greifschenkel eine Art Zange bilden, mit der ein Filter gehalten werden kann. Dies kann insbesondere durch Reibung erfolgen. Filter und Filterhalter können jedoch ergänzend oder alternativ auch so zueinander ausgeformt sein, dass zum Halten ein Formschluss genutzt wird.

An dem drehbar gelagerten ersten Greifschenkel kann beispielsweise eine Federkraft anliegen, die in eine zangenmäßige Bewegung des ersten Greifschenkels in Richtung des zweiten Greifschenkels umsetzbar ist. Um diese Federkraft zu erzeugen und entsprechend umzusetzen, kann der zweite Greifschenkel beispielsweise an einer Achse montiert sein, auf welcher wiederum eine Gleitlagerbuchse axial beweglich gelagert ist. Dabei ist die Gleitlagerbuchse federbelastet, wodurch eine permanente Druckkraft auf einen Bereich des ersten Greifschenkels wirkt, der so ausgeformt ist, dass die Druckkraft in eine zangenmäßige Bewegung des ersten Greifschenkels in Richtung des zweiten Greifschenkels umsetzbar ist.

Von der Erfindung umfasst ist ferner ein medizinisches Gerät zur extrakorporalen Blutbehandlung, das einen Filterhalter am Gehäuse des Geräts umfasst, wobei der Filterhalter erfindungsgemäß ausgebildet ist. Ein solches medizinisches Gerät hat insbesondere den Vorteil, dass am Gehäuse keine störenden oder anfälligen Schlauchhalter für den Bereich des Dialysators vorgesehen sein müssen, da der Filterhalter bereits die Funktion der Schlauchhalterung erfüllt.

Weitere Vorteile, Besonderheiten und zweckmäßige Weiterbildungen der Erfindung ergeben sich aus den Unteransprüchen und der nachfolgenden Darstellung bevorzugter Ausführungsbeispiele anhand der Abbildungen.

Von den Abbildungen zeigt:
- Fig. 1: eine schematische Darstellung eines Ausführungsbeispiels des erfindungsgemäßen Filterhalters;
- Fig. 2: eine schematische Darstellung des Filterhalters gemäß Fig. 1 mit eingebrachtem Filter und Schlauch.

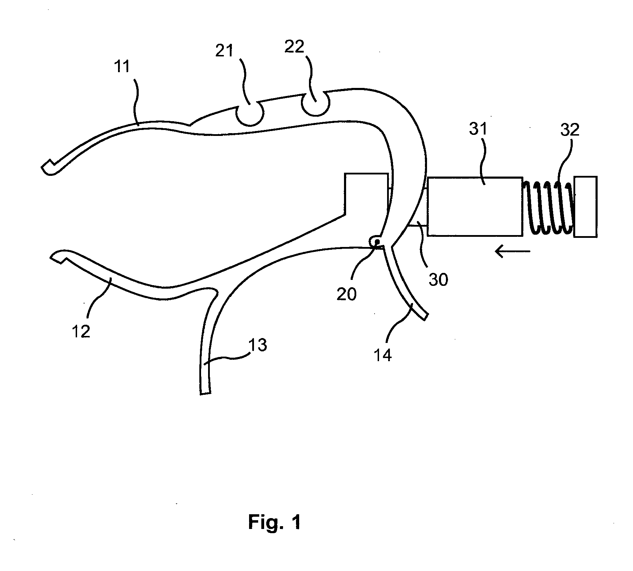

Fig. 1 zeigt ein Ausführungsbeispiel des erfindungsgemäßen Filterhalters 10 ohne eingebrachten Filter. Bei einem zu haltenden Filter kann es sich beispielsweise um den Dialysator eines medizinischen Geräts zur extrakorporalen Blutbehandlung handeln, wobei der Filterhalter 10 in diesem Fall auch als Dialysatorhalter bezeichnet wird.

Um einen Filter im Filterhalter 10 aufnehmen zu können, sind zwei Greifschenkel 11 und 12 vorgesehen, zwischen denen ein Aufnahmebereich zur Einbringung des Filters ausgebildet ist. Diese Greifschenkel können auch als Dialysatorklemmen bezeichnet werden, wenn es sich bei dem Filter um einen Dialysator handelt. Zur Aufnahme eines Filters sind die Greifschenkel 11, 12 in einem Greifbereich nach innen gebogen ausgeführt, um einen zylinderförmigen Filter gut greifen und halten zu können. Die Innenflächen der Greifschenkel 11, 12 können ferner geriffelt ausgeführt sein oder eine spezielle Oberfläche mit hohem Reibungskoeffizienten aufweisen.

Ein erster Greifschenkel 11 ist drehbar an einem Drehpunkt 20 am zweiten Greifschenkel 12 gelagert. Vorzugsweise erfolgt dies federbelastet, so dass eine Federkraft eine zangenförmige Bewegung des ersten Greifschenkels 11 in Richtung des zweiten Greifschenkels 12 bewirkt, wodurch ein Filter zwischen den beiden Greifschenkeln 11, 12 eingeklemmt werden kann. Die Federkraft wird beispielsweise durch eine Gleitlagerbuchse 31 aufgebracht, die axial beweglich auf einer Achse 30 gelagert ist, die wiederum fest mit dem zweiten Greifschenkel 12 verbunden ist. Auf die Gleitlagerbuchse 31 wirkt durch eine Druckfeder 32 eine permanente Kraft nach links, wie es in Fig. 1 durch einen Pfeil dargestellt ist. Durch diese Federkraft wirkt eine permanente Druckkraft auf die Außenkontur des ersten Greifschenkels 11, der an dieser Stelle so gewölbt ausgeformt ist, dass diese Druckkraft in eine zangenförmige Bewegung des ersten Greifschenkels 11 in Richtung des zweiten Greifschenkels 12 umsetzbar ist.

Am ersten Greifschenkel 11 ist ferner im Bereich des Drehpunkts 20 ein erster Spreizgriff 14 ausgebildet, während am zweiten Greifschenkel 12 ein zweiter Spreizgriff 13 ausgebildet ist. Die beiden Spreizgriffe 13, 14 sind dabei so ausgeformt und angeordnet, dass sie gleichzeitig von einem Bediener gegriffen und aufeinander zugezogen werden können. Durch diese Bewegung öffnet sich der Filterhalter 10, indem der erste Greifschenkel 11 bezüglich des zweiten Greifschenkels 12 um den Drehpunkt 20 nach außen geschwenkt wird. Hierdurch wird der Abstand zwischen den beiden Greifschenkeln 11, 12 vergrößert und ein Filter kann in den Aufnahmebereich zwischen den beiden Greifschenkeln 11, 12 eingebracht werden.

Am ersten Greifschenkel 11 ist wenigstens eine rinnenförmige Einbuchtung in das Profil des Greifschenkels 11 eingebracht, wobei in dem in den Figuren dargestellten Ausführungsbeispiel zwei derartige Einbuchtungen 21 und 22 vorgesehen sind. Diese Einbuchtungen 21, 22 bilden am Greifschenkel 11 Schlauchhalter aus, in welche Schläuche einklemmbar sind. Die Schlauchhalter liegen dabei vorzugsweise an der Außenseite des Greifschenkels 11, sie können jedoch beispielsweise auch an der Innenseite angebracht sein. Darüber hinaus können Schlauchhalter auch am zweiten Greifschenkel 12 ausgeformt sein.

Ferner haben die rinnenförmigen Einbuchtungen 21, 22 vorzugsweise einen im Wesentlichen runden Querschnitt, es können jedoch auch beliebige andere Querschnitte gewählt werden, solange sie für eine klemmende Einbringung von Schläuchen geeignet sind. Hierzu sollten an der Grenzfläche zwischen einem Greifschenkel und der Innenfläche der Einbuchtung Kanten vorgesehen sein, welche die Öffnung des Schlauchhalters soweit verkleinern, dass ein Schlauch bei der Einbringung leicht gequetscht wird. So wird der Schlauch innerhalb der Einbuchtung eingeklemmt und gehalten.

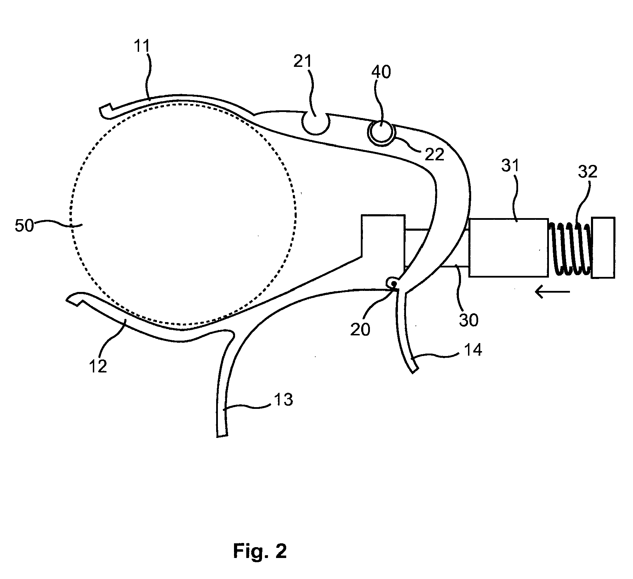

Fig. 2 zeigt eine schematische Darstellung eines Ausführungsbeispiels des erfindungsgemäßen Filterhalters 10 mit einem eingebrachten Filter 50, dessen Querschnitt gestrichelt angedeutet ist. Dazu wurden die Greifschenkel 11, 12 des Filterhalters 10 auseinander gespreizt, so dass der Filter 50 von links in den Aufnahmebereich zwischen den beiden Greifschenkeln 11, 12 eingeschoben werden konnte. Für diesen Vorgang sind die vorderen Enden der beiden Greifschenkel beispielsweise leicht nach außen gewölbt ausgeführt, um eine Führung des Filters mit rundem Querschnitt in den Aufnahmebereich zu erleichtern.

Durch die Schwenkbewegung des ersten Greifschenkels 11 wurde die Gleitlagerbuchse 31 nach rechts auf der Achse 30 verschoben, so dass sich die Druckkraft durch die Feder 32 auf die Gleitlagerbuchse 31 erhöht hat. Der Filter 50 kann so zwischen den beiden Greifschenkeln 11, 12 eingeklemmt werden. Ergänzend kann der Filter 50 auch so ausgeformt sein, dass er beispielsweise eine umlaufende Ausbuchtung aufweist, mit welcher er auf der Oberseite der Greifschenkel 11, 12 aufliegt und so gegen ein Herausrutschen nach unten gesichert ist.

Ferner ist in Fig. 2 gezeigt, wie ein Schlauch 40 in die Einbuchtung 22 eingebracht ist. Hierbei verläuft die Einbuchtung 22 quer zur Längsausrichtung des Greifschenkels 11, so dass der Schlauch 40 parallel zur Längsachse des Filters 50 am Filterhalter 10 ausgerichtet ist.

### Bezugszeichenliste

- 10: Filterhalter, Dialysatorhalter
- 11: Erster Greifschenkel, Dialysatorklemme
- 12: Zweiter Greifschenkel, Dialysatorklemme
- 13,14: Spreizgriff
- 20: Drehpunkt
- 21,22: Einbuchtung, Schlauchhalter
- 30: Achse
- 31: Gleitlagerbuchse
- 32: Feder, Druckfeder
- 40: Schlauch
- 50: Filter

## Patentansprüche

1. Filterhalter (10) zur Halterung eines Filters (50) am Gehäuse eines medizinischen Geräts zur extrakorporalen Blutbehandlung, wobei der Filterhalter (10) wenigstens einen ersten Greifschenkel (11) und einen zweiten Greifschenkel (12) aufweist, die so zueinander angeordnet sind, dass zwischen den beiden Greifschenkeln (11;12) ein Aufnahmebereich gebildet ist, und der Filter (50) so in diesen Aufnahmebereich zwischen den beiden Greifschenkeln (11;12) einbringbar ist, dass der Filter (50) wenigstens gegen die Schwerkraft im Filterhalter (10) gehalten wird, **dadurch gekennzeichnet, dass** an wenigstens einem Greifschenkel (11;12) wenigstens eine rinnenförmige Einbuchtung (21;22) vorgesehen ist, deren Querschnitt das Einklemmen eines Schlauchs (40) ermöglicht.

2. Filterhalter nach Anspruch 1,
**dadurch gekennzeichnet, dass** sich die wenigstens eine rinnenförmige Einbuchtung (21;22) an einer Außenseite des Greifschenkel (11;12) befindet, die von dem Aufnahmebereich weg gerichtet ist.

3. Filterhalter nach einem oder beiden der Ansprüche 1 und 2,
**dadurch gekennzeichnet, dass** an dem wenigstens einen Greifschenkel (11;12) zwei rinnenförmige Einbuchtungen (21;22) vorgesehen sind.

4. Filterhalter nach einem oder mehreren der Ansprüche 1 bis 3,
**dadurch gekennzeichnet, dass** der wenigstens eine Greifschenkel (11;12) aus einem Metallprofil gebildet ist.

5. Filterhalter nach Anspruch 4,
**dadurch gekennzeichnet, dass** das Metallprofil ein Strangpressprofil ist.

6. Filterhalter nach einem oder mehreren der Ansprüche 1 bis 5,
**dadurch gekennzeichnet, dass** die wenigstens eine rinnenförmige Einbuchtung (21;22) quer zur Längsausrichtung eines Greifelements (11;12) verläuft.

7. Filterhalter nach einem oder mehreren der Ansprüche 1 bis 6,
**dadurch gekennzeichnet, dass** der erste Greifschenkel (11) drehbar an dem zweiten Greifschenkel (12) gelagert ist.

8. Filterhalter nach Anspruch 7,
**dadurch gekennzeichnet, dass** an dem drehbar gelagerten ersten Greifschenkel (11) eine Federkraft anliegt, die in eine zangenmäßige Bewegung des ersten Greifschenkels (11) in Richtung des zweiten Greifschenkels (12) umsetzbar ist.

9. Filterhalter nach Anspruch 8,
**dadurch gekennzeichnet, dass** der zweite Greifschenkel (12) an einer Achse (30) montiert ist, auf welcher eine Gleitlagerbuchse (31) axial beweglich gelagert ist, wobei die Gleitlagerbuchse (31) federbelastet ist, wodurch eine permanente Druckkraft auf einen Bereich des ersten Greifschenkels (11) wirkt, der so ausgeformt ist, dass die Druckkraft in eine zangenmäßige Bewegung des ersten Greifschenkels (11) in Richtung des zweiten Greifschenkels (12) umsetzbar ist.

10. Medizinisches Gerät zur extrakorporalen Blutbehandlung, umfassend einen Filterhalter (10) am Gehäuse des Geräts,
**dadurch gekennzeichnet, dass** der Filterhalter (10) gemäß einem der Ansprüche 1 bis 9 ausgebildet ist.

## Claims

1. Filter holder (10) for the support of a filter (50) on the casing of a medical device for extra-corporal blood treatment, wherein the filter holder (10) has at least a first grip leg (11) and a second grip leg (12), which are arranged with respect to each other such that a reception area is formed between the two grip legs (11;12), and the filter (50) is capable of being brought into this reception area between the two grip legs (11;12), so that the filter (50) is held in the filter holder (10) against the force of gravity at least
**characterized in that** on at least one grip leg (11;12) at least one trough-shaped indentation or concavity (21;22) is provided, whose cross-section is adapted for wedging in of a hose (40).

2. Filter holder according to Claim 1,
**characterized in that** the at least one trough-shaped indentation or concavity (21;22) is located on an outer side of the grip leg (11;12), which outer side is directed away from the reception area.

3. Filter holder according to one or both of the Claims 1 and 2,
**characterized in that** two trough-shaped indentations or concavities (21;22) are provided on the at least one grip leg (11;12).

4. Filter holder according to one or more of the Claims 1 to 3,
**characterized in that** the at least one grip leg (11;12) is formed from a metal profile.

5. Filter holder according to Claim 4,
**characterized in that** the metal profile is an extruded section.

6. Filter holder according to one or more of the Claims 1 to 5,
**characterized in that** the at least one trough-shaped indentation or concavity (21;22) extends perpendicular to the longitudinal alignment of a grip element (11;12).

7. Filter holder according to one or more of the Claims 1 to 6,
**characterized in that** the first grip leg (11) is pivotingly supported on the second grip leg (12).

8. Filter holder according to Claim 7,
**characterized in that** a spring force is applied on the first grip leg (11) which is pivotingly supported, which spring force can be converted into a forceps-type movement of the first grip leg (11) in the direction to the second grip leg (12).

9. Filter holder according to Claim 8,
**characterized in that** the second grip leg (12) is assembled on an axle (30), on which a plain bearing bush (31) is supported axially movable, wherein the plain bearing bush (31) is spring-loaded, wherein a permanent pressure force acts on an area of the first grip leg (11), which is formed such that the pressure force can be converted into a forceps-type movement of the first grip leg (11) in the direction to the second grip leg (12).

10. Medical device for extra-corporal blood treatment, including a filter holder (10) on the casing of the device,
**characterized in that** the filter holder (10) is formed in accordance with one of the Claims 1 to 9.

## Revendications

1. Porte-filtre (10) pour supporter un filtre (50) sur le boitier d'un appareil médical pour le traitement extracorporel du sang, le porte-filtre (10) présentant au moins une première branche de préhension (11) et une deuxième branche de préhension (12), qui sont agencées l'une par rapport à l'autre de manière à former une zone d'accueil entre les deux branches de préhension (11; 12), et le filtre (50) pouvant être inséré dans cette zone d'accueil entre les deux branches de préhension (11; 12) de façon telle, que le filtre (50) soit maintenu, au moins à l'encontre de la force de gravité, dans le porte-filtre (10),
**caractérisé en ce que** sur au moins une branche de préhension (11; 12) est prévue au moins une encoche en forme de goulotte (21; 22) dont la section transversale permet le serrage d'un tuyau ou d'une tubulure (40).

2. Porte-filtre selon la revendication 1,
**caractérisé en ce que** ladite au moins une encoche en forme de goulotte (21; 22) se trouve sur un côté extérieur de la branche de préhension (11; 12), qui pointe dans la direction s'éloignant de la zone d'accueil.

3. Porte-filtre selon l'une ou les deux revendications 1 et 2,
**caractérisé en ce que** sur ladite au moins une branche de préhension (11; 12) sont prévues deux encoches en forme de goulotte (21; 22).

4. Porte-filtre selon l'une ou plusieurs des revendications 1 à 3,
**caractérisé en ce que** ladite au moins une branche de préhension (11; 12) est formée par un profilé métallique.

5. Porte-filtre selon la revendication 4,
**caractérisé en ce que** le profilé métallique est un profilé filé.

6. Porte-filtre selon l'une ou plusieurs des revendications 1 à 5,
**caractérisé en ce que** ladite au moins une encoche en forme de goulotte (21; 22) s'étend transversalement à l'orientation longitudinale d'un élément de préhension (11; 12).

7. Porte-filtre selon l'une ou plusieurs des revendications 1 à 6,
**caractérisé en ce que** la première branche de préhension (11) est montée rotative sur la deuxième branche de préhension (12).

8. Porte-filtre selon la revendication 7,
**caractérisé en ce qu'**une force de ressort est appliquée sur la première branche de préhension (11) montée rotative, et peut être convertie en un mouvement, similaire à celui d'une pince, de la première branche de préhension (11) en direction de la deuxième branche de préhension (12).

9. Porte-filtre selon la revendication 8,
**caractérisé en ce que** la deuxième branche de préhension (12) est montée sur un axe (30) sur lequel est placé de manière axialement mobile, une douille lisse coulissante (31), la douille lisse coulissante (31) étant sollicitée par ressort, ce qui produit l'action permanente d'une force de pression sur une zone de la première branche de préhension (11), qui est d'une configuration telle que la force de pression puisse être convertie en un mouvement, similaire à celui d'une pince, de la première branche de préhension (11) en direction de la deuxième branche de préhension (12).

10. Appareil médical pour le traitement extracorporel du sang, comprenant un porte-filtre (10) sur le boitier de l'appareil,
**caractérisé en ce que** le porte-filtre (10) est réalisé conformément à l'une des revendications 1 à 9.
